(19) Europäisches Patentamt
European Patent Office
Office européen des brevets

(11) Numéro de publication : **0 441 718 A1**

(12) **DEMANDE DE BREVET EUROPEEN**

(21) Numéro de dépôt : **91420024.1**

(22) Date de dépôt : **28.01.91**

(51) Int. Cl.$^5$ : **C07D 471/04,** C07D 213/77, C07D 257/04, C07D 419/04, A01N 43/90, // (C07D471/04, 249:00, 221:00)

(30) Priorité : **29.01.90 FR 9001267**

(43) Date de publication de la demande : **14.08.91 Bulletin 91/33**

(84) Etats contractants désignés : **AT BE CH DE DK ES FR GB GR IT LI LU NL SE**

(71) Demandeur : **RHONE-POULENC AGROCHIMIE
14-20, rue Pierre Baizet
F-69009 Lyon (FR)**

(72) Inventeur : **Peignier, Raymond
8Ibis chemin de Vassieux
F-69300 Caluire (FR)**
Inventeur : **Chene, Alain
Les Terrasses de Saint Rambert
12, chemin de Montpellas F-69009 Lyon (FR)**
Inventeur : **Cantegril, Richard
31 rue de Lattre de Tassigny
F-69009 Lyon (FR)**
Inventeur : **Mortier, Jacques
37 rue Louis Bouquet
F-69009 Lyon (FR)**

(74) Mandataire : **Brachotte, Charles et al
RHONE-POULENC AGROCHIMIE DPI 14, 20
Rue Pierre Baizet
F-69009 Lyon (FR)**

(54) **Composés herbicides à groupe triazolopyridine.**

(57) Composés utilisables comme herbicides et ayant la formule (I)

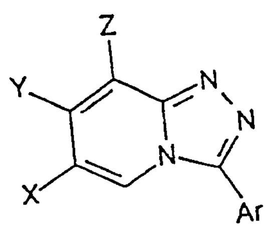

Formule (I)

dans laquelle :

* X , Y et Z représentent un atome d'hydrogène ou un atome d'halogène ou un groupe alkyle ou haloalkyle ou alkoxy, l'un au moins des radicaux X, Y ou Z ayant une signification autre que l'atome d'hydrogène,

* Ar représente un groupe phényle, éventuellement mono- ou poly- substitué par un groupe alkyle inférieur ou alkoxy inférieur ou alkylthio inférieur ou phényle ou phénoxy ou un atome d'halogène, ou un hétérocycle Het,

* Het représente un hétérocycle à 5 ou 6 maillons et 1 ou plusieurs hétéroatome(s) tel(s) que le soufre, l'azote ou l'oxygène, cet hétérocycle étant eventuellement mono- ou poly-substitué par un groupe alkyle inférieur ou alkoxy inférieur ou alkylthio inférieur ou un atome d'halogène.

Les compositions herbicides contenant les produits de formule (I) et les procédés de désherbage mettant en oeuvre les composés de formule (I) ou les compositions les contenant.

EP 0 441 718 A1

# COMPOSES HERBICIDES A GROUPE TRIAZOLOPYRIDINE

La présente invention concerne ainsi de nouveaux composés herbicides de la famille des 1,2,4-triazolo-[4,3-a] pyridines, ainsi que leurs procédés de préparation, les compositions les contenant et leur utilisation pour la lutte contre les mauvaises herbes.

Un objet de la présente invention est donc de proposer des composés utiles à la fois en préémergence (ou prélevée) et à la fois en postémergence (ou postlevée) comme herbicides.

Un autre objet de la présente invention est de proposer des composés utiles à la fois contre les mauvaises herbes de type monocotylédones et celles de type dicotylédones.

Un autre objet de la présente invention est de proposer des composés utiles en pré- et/ou post-émergence comme herbicides sélectifs de cultures monocotylédones (notamment le blé, le maïs et le riz) et de cultures dicotylédones (notamment le soja, le coton, le tournesol).

Il a maintenant été trouvé que tout ou partie de ces buts pouvaient être atteints au moyen des nouveaux composés selon l'invention.

Dans le présent exposé, par le terme inférieur qualifiant un radical, on entend que ce radical peut avoir au plus 4 atomes de carbone.

Les composés selon l'invention sont caractérisés en ce qu'ils ont pour formule la formule (I) indiquée en fin de description, dans laquelle :

* X, Y et Z représentent un atome d'hydrogène ou un atome d'halogène ou un groupe alkyle ou haloalkyle ou alkoxy, l'un au moins des radicaux X, Y ou Z ayant une signification autre que l'atome d'hydrogène,

* Ar représente un groupe phényle, éventuellement mono- ou poly- substitué (de préférence monosubstitué) par un groupe alkyle inférieur ou alkoxy inférieur ou alkylthio inférieur ou phényle ou phenoxy ou un atome d'halogène, de préférence le chlore ou le fluor ; ou un hétérocycle Het,

* Het représente un hétérocycle à 5 ou 6 maillons et contenant un ou plusieurs hétéroatome(s) tel(s) que le soufre, l'azote ou l'oxygène, cet hétérocycle étant éventuellement mono- ou poly-substitué (de préférence monosubstitué) par un groupe alkyle inférieur ou alkoxy inférieur ou alkylthio inférieur ou un atome d'halogène, de préférence le chlore ou le fluor,

* à l'exclusion des composés tels que

** simultanément Y représente un groupe méthyle, X et Z représentent l'atome d'hydrogène, Ar représente un groupe 3-chlorophényle ou 4-chlorophényle ou 3-pyridyle ou 3,4,5-triméthoxyphényle ; ou bien

** simultanément X représente l'atome de chlore, Y et Z représentent l'atome d'hydrogène, Ar représente un groupe 2-pyridyle ou 4-pyridyle.

L'invention concerne également les sels (acceptables en agriculture) des dérivés précédents, spécialement les sels d'addition avec un acide, cet acide pouvant être minéral ou organique comme par exemple les acides chlorhydrique, sulfurique, acétique, arylsulfonique.

Parmi les composés selon l'invention, on préfère les composés ayant l'une ou l'autre des caractéristiques suivantes :

un seul ou deux des trois radicaux X, Y ou Z représente un radical autre que l'atome d'hydrogène

lorsque X, Y ou Z sont un atome d'halogène, il s'agit de l'atome de chlore ou de brome

lorsque X, Y ou Z sont un radical au moins partiellement hydrocarboné, ce radical a de préférence de 1 à 4 atomes de carbone, et plus préférentiellement encore 1 atome de carbone

X est autre que l'atome de chlore

Z est autre que l'atome d'hydrogène, avantageusement un atome de chlore ou un groupe méthyle

X et Y sont l'atome d'hydrogène (et Z est autre que l'atome d'hydrogène)

lorsque Ar représente un groupe phényle substitué, les substituants se trouvent en position ortho ou para.

Comme groupe Het particulièrement utilisables dans l'invention, on peut citer les groupes thiényles (de préférence 2- et 3- thiényle), thiazolyles (de préférence 2- et 4- thiazolyle), pyridyles (de préférence 2-pyridyle), pyrrolyles (de préférence 2-pyrrolyle), thiadiazolyles (de préférence 5-thiadiazolyles).

Comme composés particuliers pouvant être réalisés selon l'invention, on peut citer les composés suivants (voir en fin de description l'indication de numérotation des atomes) :

8-chloro 3-(3'-méthyl thién-2'-yl) -s-triazolo [4,3-a] pyridine,

8-chloro 3-phényl -s-triazolo [4,3-a] pyridine,

8-méthyl 3-(3'-méthyl thién-2'-yl) -s-triazolo [4,3-a] pyridine,

8-méthyl 3-(thién-3'-yl) -s-triazolo [4,3-a] pyridine,

8-méthyl 3-(pyrid-2'-yl) -s-triazolo [4,3-a] pyridine,

8-méthyl 3-(4'-méthyl phényl) -s-triazolo [4,3-a] pyridine,

8-méthyl 3-(4′-fluoro phényl) -s-triazolo [4,3-a] pyridine,
8-méthyl 3-(2′-chloro phényl) -s-triazolo [4,3-a] pyridine,
6-méthyl 3-phényl -s-triazolo [4,3-a] pyridine,
8-trifluorométhyl 3-phényl -s-triazolo [4,3-a] pyridine,
6-chloro 3-phényl -s-triazolo [4,3-a] pyridine,
8-méthyl 3-(pyrid-3′-yl) -s-triazolo [4,3-a] pyridine,
8-méthyl 3-(pyrid-4′-yl) -s-triazolo [4,3-a] pyridine,
8-méthyl 3-(furan-2′-yl) -s-triazolo [4,3-a] pyridine,
8-méthyl 3-(4′-méthoxy phényl) -s-triazolo [4,3-a] pyridine,
7-méthyl 3- phényl -s-triazolo [4,3-a] pyridine,
8-méthyl 3-(3′-méthyl phényl) -s-triazolo [4,3-a] pyridine,
8-méthyl 3-(2′,4′-dichloro phényl) -s-triazolo [4,3-a] pyridine,
8-méthyl 3-(4′-chloro phényl) -s-triazolo [4,3-a] pyridine,
8-méthyl 3-(4′-phényl phényl) -s-triazolo [4,3-a] pyridine,
8-méthoxy 3- phényl -s-triazolo [4,3-a] pyridine,
8-méthyl 3-(thién-2′-yl) -s-triazolo [4,3-a] pyridine,
8-méthyl 3-(4′-thiométhyl phényl) -s-triazolo [4,3-a] pyridine,
8-méthyl 3-(2′-fluoro phényl) -s-triazolo [4,3-a] pyridine,
8-méthyl 3-(2′-méthyl phényl) -s-triazolo [4,3-a] pyridine,
8-méthyl 3-(2′,4′-diméthyl phényl) -s-triazolo [4,3-a] pyridine,
8-méthyl 3-phényl -s-triazolo [4,3-a] pyridine.
8-bromo, 3-[3′-méthyl thién-2′yl]-s-triazolo [4,3-a] pyridine,
8-trifluorométhyl, 3-[3′-méthyl thién-2′yl]-s-triazolo [4,3-a] pyridine,
8-méthyl, 3-[2′-méthyl thién-3′yl]-s-triazolo [4,3-a] pyridine,
8-bromo, 3-phényl-s-triazolo [4,3-a] pyridine,
8-chloro, 3-[2′-méthyl thién-3′yl]-s-triazolo [4,3-a] pyridine,
8-chloro, 3-[thién-3′yl]-s-triazolo [4,3-a] pyridine,
8-méthyl, 3-[1′-méthyl pyrrol-2′yl]-s-triazolo [4,3-a] pyridine,
8-chloro, 3-[3′,5′-diméthyl thién-2′yl]-s-triazolo [4,3-a] pyridine,
7,8-Diméthyl,3-Phényl-s triazolo-[4,3-a] pyridine,
8-méthyl, 3-[3′,5′-diméthyl thién-2′yl]-s-triazolo [4,3-a] pyridine,
8-éthyl, 3-[3′-méthyl thién-2′yl]-s-triazolo [4,3-a] pyridine,
8-éthyl, 3-phényl-s-triazolo [4,3-a] pyridine,
8-éthyl, 3-[1′-méthyl pyrrol-2′yl]-s-triazolo [4,3-a] pyridine,
8-éthyl, 3-[thién-3′yl]-s-triazolo [4,3-a] pyridine,
8-trifluorométhyl, 3-[1′-méthyl pyrrol-2′yl]-s-triazolo [4,3-a] pyridine,
8-trifluorométhyl, 3-(pyryd-2′yl]-s-triazolo [4,3-a] pyridine,
8-chloro, 3-[4′-méthyl thién-2′yl]-s-triazolo [4,3-a] pyridine,
7,8-Diméthyl,3-[3′-méthyl thién-2′yl]-s-triazolo [4,3-a] pyridine,
8-méthyl, 3-[4′-méthyl thién-2′yl]-s-triazolo [4,3-a] pyridine,
8-trifluorométhyl, 3-[thièn -3′yl]-s-triazolo [4,3-a] pyridine,
8-méthyl, 3-[4′-isopropyl phényl]-s-triazolo [4,3-a] pyridine,
8-méthyl, 3-[4′-bromo thièn-2′yl]-s-triazolo [4,3-a] pyridine,
8-chloro, 3-[4′-bromo thièn-2′yl]-s-triazolo [4,3-a] pyridine,

Les composés selon l'invention peuvent être préparés au moyen de divers procédés.

Selon un premier procédé de préparation de composés de formule (I), on fait réagir un composé de type arylidène 2-(pyrid-2′-yl)hydrazine ayant pour formule la formule (II), dans laquelle les divers substituants ont les significations données précédemment, avec un agent d'oxydation, selon une réaction d'oxydation cyclisante. Comme agent d'oxydation, on peut citer les cations métalliques dérivés de métaux ayant plusieurs degrés d'oxydation et se trouvant à un degré d'oxydation supérieur, comme par exemple le tétraacétate de plomb ou le chlorure ferrique ; on peut aussi utiliser l'oxygène de l'air comme agent d'oxydation. La réaction s'effectue avantageusement en milieu liquide solvant organique, le solvant étant choisi de préférence de manière à dissoudre le plus possible les réactifs et les produits finaux. Des solvants convenables sont les hydrocarbures, les hydrocarbures halogénés, les acides, les alcools ; les solvants de type hydrocarbures aromatiques nitrés peuvent aussi être utilisés, spécialement dans le cas où l'agent d'oxydation est l'oxygène de l'air. Le rapport molaire de la quantité d'agent d'oxydation mis en oeuvre par rapport au composé de formule (II) est généralement compris entre 1 et 5.

Selon une première variante, la réaction d'oxydation du composé de formule (II) peut également être effectuée par addition d'un halogène (en quantité de préférence voisine de la stoechiométrie), tel que le brome, suivie d'une réaction de déshalogénation. Ces réactions d'oxydation (selon le mode général ou selon la variante) s'effectuent généralement entre 10 et 210°C (de préférence entre 10 et 50 °C pour la variante).

L'oxydation cyclisante est obtenue en faisant suivre cette oxydation par halogénation d'une réaction de déshalogénation qui s'effectue en général en présence d'agent alcalin, par exemple en présence d'un sel alcalin carboxylique tel que l'acétate de sodium en milieu acide acétique. Le rapport molaire de la quantité d'agent alcalin mis en oeuvre par rapport au composé de formule (II) halogéné est généralement compris entre 1 et 5.

Selon une deuxième variante, la réaction d'oxydation du composé de formule (II) peut également être effectuée par addition d'un N-halogéno-N-métallosulfonamidate de formule (IV) :

$RSO_2NX$ M où R représente un groupe alkyle, ou de préférence phényle éventuellement mono-substitué en para par un groupe alkyle (par exemple un groupe p-tolyle), X représente un atome d'halogène (de préférence le chlore ou le brome) et M un atome de métal alcalin (de préfrence le sodium). Un exemple préféré de N-halogéno-N-métallosulfonamidate est la chloramine T de formule (IVa) :

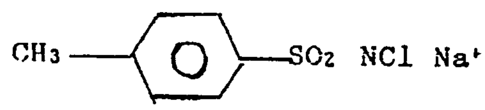

L'utilisation d'un N-halogéno-N-métallosulfonamidate de formule précédente comme agent d'oxydation cyclisante des arylidène 2-(pyrid-2'yl)hydrazines ayant pour formule la formule générale (II) est originale et constitue ainsi un nouveau procédé de préparation des composés de formule (I)

Cette réaction d'oxydation cyclisante s'effectue généralement entre 10 et 150°C (de préférence entre 20 et 50°C) et s'effectue avantageusement en milieu liquide solvant organique, de préférence les alcools.

Le rapport molaire de la quantité d'agent d'oxydation mis en oeuvre par rapport au composé de formule (II) est de préférence voisin de la stoechiométrie.

Selon une deuxième méthode de préparation de composés de formule (I), on déshydrate (déshydratation cyclisante) un composé de type 2- (aroylhydrazino)pyridine ayant pour formule la formule (III) dans laquelle les divers substitutants ont la même signification que dans la formule (I).

La réaction de déshydratation cyclisante du composé de formule (III) s'opère habituellement par chauffage entre 100 et 250 °C, avec élimination de l'eau formée au fur et à mesure du déroulement de la réaction. L'eau formée peut être éliminée par simple distillation ou bien aussi par distillation azéotropique si l'on effectue le chauffage en présence d'un solvant aromatique capable de dissoudre le composé de formule (III) et de former un azéotrope avec l'eau. Comme solvant azéotrope convenant pour ce mode de déshydratation on peut citer les hydrocarbures aromatiques, halogénés ou non, ainsi que les phénols, par exemple le xylène, le phénol, le 1,2,4-trichlorobenzène.

Selon une première variante de la déshydratation cyclisante du composé de formule (III), on opére en présence d'un agent de déshydratation, de préférence, en présence d'un solvant inerte, par exemple un hydrocarbure aromatique tel que le benzène, le toluène et le xylène. L'agent déshydratant est avantageusement un capteur d'eau connu en soi, tel que $POCl_3$ ou l'acide acétique concentré ou l'acide polyphosphorique, la quantité d'agent déshydratant mis en oeuvre étant avantageusement comprise entre 1 et 50 fois la quantité en moles du composé de formule (III).

Selon une seconde variante, la cyclisation déshydratante des composés de formule (III) s'effectue avec passage transitoire par un composé intermédiaire de formule (XI) ou (XII) dans lesquelles les substituants ont les mêmes significations que dans la formule (I).

Les composés intermédiaires de formule (XI) se forment par action de $SOCl_2$ sur un composé de formule (III) [en quantité par exemple comprise entre 1 à 3 moles par mole de composé de formule (III)], à température comprise entre -5°C et 50 °C, de préférence à température ambiante ; la réaction s'effectue avantageusement en présence d'un solvant et/ou d'un capteur d'acide, notamment la pyridine ou le diméthylformamide (DMF) en présence de triéthylamine.

La thermolyse des composés de formule (XI) en composés de formule (I) s'accompagne d'un dégagement de $SO_2$. Cette thermolyse est faite préférentiellement en présence d'un solvant, tel que les nitriles ou les hydrocarbures aromatiques, notamment l'acétonitrile, le toluène, les xylènes ou les naphtalènes éventuellement alkylés (de préférence méthylés).

La déshydratation cyclisante des composés de formule (III) en composés de formule (I), en passant par des composés intermédiaires de formule (XII), s'effectue avantageusement par action de composés de type dichlorotriarylphosphoranes sur un composé de formule (III), en présence de base tertiaire telle que la triéthylamine et de solvant de type nitrile.

Selon une troisième méthode de préparation de composés de formule (I), on opère une désamination cyclisante par chauffage d'un composé de type amidrazone ayant pour formule (IV) dans laquelle les divers substituants ont même signification que dans la formule (I).

La réaction s'effectue avantageusement en présence d'agents (liquides) de capture de la molécule d'ammoniac, par exemple en présence d'acides, d'anhydrides ou d'halogénures d'acides, ces acides étant de préférence des acides carboxyliques. La température est généralement comprise entre 25 et 220°C, de préférence entre 50 et 180°C. La quantité d'agent de capture de la molécule d'ammoniac est généralement comprise entre 1 et 30 fois la quantité (en moles) du produit de formule (IV).

On peut également opérer la désamination par simple chauffage en milieu liquide (désamination thermique), de préférence en milieu solvant organique inerte, par exemple un hydrocarbure aromatique halogéné ou non.

Selon un quatrième procédé de préparation, des produits de formule (I), on fait réagir un composé de type hydrazine de formule (V) avec un composé de formule (VI) : $U_1$-C(=$W_1$)-Ar selon la réaction :

$$(V) + (VI) \rightarrow (I) + U_1H + W_1H_2$$

les divers radicaux de ces formules (V) et (VI) ayant les mêmes significations que dans la formule (I) et, en outre :

$W_1$ représente un atome d'oxygène ou un groupe NH,

lorsque $W_1$ représente l'atome d'oxygène, alors $U_1$ représente un groupe hydroxyle [(VI) est alors un acide] ou alkoxy [(VI) est alors un ester] ou aroyloxy [(VI) est alors un anhydride d'acide], ou un atome d'halogène [(VI) est alors un halogénure d'acide], de préférence le chlore,

lorsque $W_1$ représente un groupe NH, alors $U_1$ représente un groupe alkoxy ou alkylthio ou arylalkylthio.

La réaction précédente s'effectue à température généralement comprise entre 20 et 200°C, de préférence entre 50 et 180°C pendant une durée de 1 à 24 heures. Les réactifs (V) et (VI) sont mis en oeuvre en proportions molaires respectives comprises entre 0,8 et 1,2 de préférence égale ou voisine de 1. La réaction peut être effectuée en présence ou en absence de solvant. Comme solvant on peut utiliser, comme indiqué ci-après dans certains cas, des solvants conduisant à la formation de mélanges azéotropiques. On peut encore utiliser des solvants de type alcools, lorsque le composé de formule (VI) est un imidate, ou un solvant aromatique tel qu'un hydrocarbure, halogéné ou non, ou la pyridine lorsque le composé de formule (VI) est un thioimidate ou un halogénure d'acide.

Les produits légers de la réaction ($U_1H$ et $W_1H_2$) sont avantageusement éliminés au fur et à mesure du déroulement de la réaction, généralement par distillation lorsqu'il s'agit d'eau ou d'alcools ($U_1$ représente alors un groupe hydroxy ou alkoxy), ou encore par capture à l'aide de base tertiaire, telle que la triéthylamine, lorsque $U_1$ est un atome d'halogène, ou encore par capture de la molécule d'ammoniac, lorsque $W_1$ est NH et $U_1$ est alkoxy (le produit de formule (VI) est alors un imidate).

La distillation, mentionnée ci-avant, de produits légers peut être une distillation azéotropique en présence de solvants aromatiques halogénés ou non, tels que la pyridine, ou les chlorobenzènes, notamment le 1,2,4-trichlorobenzène.

La capture d'amoniac, mentionnée ci-avant, est effectuée avantageusement en présence d'acides (de préférence carboxyliques) ou de leurs dérivés, tels que les anhydrides ou les halogénures d'acides. Ces acides, ou leurs dérivés, sont utilisés en quantité molaire généralement comprise entre 1 et 30 fois la quantité de produit de formule (VI) mis en oeuvre. Ces proportions sont du même ordre de grandeur (mutatis mutandis) lorsqu'on met en oeuvre une base lorsque $U_1$ est un atome d'halogène.

Selon un cinquième procédé de préparation de composés de formule (I), on fait réagir un dérivé pyridinique de formule (VIII) avec un composé tétrazolique de formule (IX), formules dans lesquelles X, Y, Z et Ar ont la même signification que dans la formule (I) et T représente un atome d'halogène, de préférence le chlore. La réaction s'effectue avantageusement entre 20 et 150°C, de préférence dans un solvant organique, par exemple un hydrocarbure aromatique, halogéné ou non, tel que les xylènes et les chlorobenzènes ou la tétraline, ou un solvant de type hétérocyclique tel que la pyridine.

L'invention concerne encore des produits nouveaux utilisables éventuellement à titre d'intermédiaires dans les procédés selon l'invention, caractérisés en ce qu'ils ont pour formule l'une des formules (II), (IV), (VIII), (XI) ou (XII) dans lesquelles les divers symboles X, Y, Z et Ar ont les significations indiquées, dans ce qui précède, pour la formule (I).

L'invention a également pour objet l'utilisation à titre d'herbicide des composés de formule (II). Dans ce qui suit, mais aussi dans ce qui précède, les réactions effectuées à chaud dans un solvant, sous réserve d'indications spécifiques contraires, sont mis en oeuvre avantageusement à la température d'ébullition du solvant considéré.

La préparation des tétrazoles de formule (IX) s'effectue commodément par réaction d'un nitrile aromatique de formule Ar-CN avec un azoture alcalin, par exemple de sodium ou d'ammonium, à des tempéra- tures

comprises entre 80 et 130°C. La quantité molaire d'azoture est avantageusement comprise entre 1 et 3 fois (de préférence 1,5 et 2 fois) la quantité de nitrile. Cette réaction s'effectue généralement dans un solvant polaire tel que le DMF ou l'acide acétique ou les alcools ou leurs mélanges. Une telle réaction est décrite dans Advances in Heterocyclic chemistry, vol. 21, pages 323-435, édité en 1977 par Academic Press dans un article de R.N.Butler intitulé : Recent advances in tetrazole chemistry.

La préparation des arylidène 2-(pyrid-2'yl) hydrazines de formule (II) s'effectue commodément par réaction de 2-hydrazinopyridines de formule (V) avec des aldéhydes de formule Ar-CHO dans lesquelles les symboles X, Y, Z et Ar ont les significations indiquées pour la formule (I). La réaction s'effectue généralement à température comprise entre 50 et 150°C, de préférence dans un solvant. Comme solvant on utilise avantageusement un alcool inférieur tel que le méthanol ou l'éthanol. La réaction est favorisée par la présence de quantités catalytiques d'un acide minéral ou organique, par exemple les acides chlorhydrique, sulfurique, acétique, trichloracétique ou perchlorique. Une telle réaction est décrite dans Quaterly review, Chemical society, vol. 23, pages 37-56, 1969 dans un article de J Buckingham ainsi que dans Houben-Weyl, Methoden der organischen Chemie, 4° édition, 1967, Vol. X-2, pages 410-487.

La préparation des 2-(aroylhydrazino) pyridines de formule (III) s'effectue commodément par réaction de 2-hydrazinopyridines de formule (V) avec des composés de formule Ar-CO-$U_2$ dans lesquelles les symboles X, Y, Z et Ar ont les significations indiquées pour la formule (I), et $U_2$ a l'une des significations indiquées précédemment pour $U_1$. La réaction se déroule selon le schéma :

$$(V) + Ar\text{-}CO\text{-}U_2 \rightarrow (III) + U_2H$$

La réaction s'effectue généralement par mélange des réactifs à température comprise entre 0 et 180°C, en présence ou en absence de solvant. Comme solvant on utilise avantageusement un solvant polaire.

Comme solvants plus particulièrement utilisables on peut donc citer les alcools lorsque Ar-CO-$U_2$ n'est pas un halogénure d'acide ; les éthers ou les hydrocarbures aliphatiques chlorés ou non, tels que le chlorure de méthylène, le chloroforme ; les solvants à propriétés capteurs d'acide, tels que la pyridine sont également utilisables lorsque Ar-CO-$U_2$ représente un halogénure d'acide.

La proportion des deux réactifs [celui de formule (V) et celui de formule Ar-CO-$U_2$] peut varier dans de larges limites autour de la stoechiométrie. Lorsque Ar-CO-$U_2$ est un acide ($U_2$ est OH) on met en oeuvre un excès de ce dernier dérivé par rapport au composé de formule (V), par exemple de 2 à 8 moles par mole de (V). Lorsque Ar-CO-$U_2$ est un ester ($U_2$ est alkoxy) on met en oeuvre une quantité, proche de la stoechiométrie, de ce dernier dérivé par rapport au composé de formule (V), par exemple de 0,8 à 1,1 mole par mole de (V). Lorsque Ar-CO-$U_2$ est un halogénure d'acide ($U_2$ est un atome d'halogène) on met en oeuvre un excès de composé de formule (V) par rapport au composé de formule Ar-CO-$U_2$, par exemple de 1 à 5 moles par mole de Ar-CO-$U_2$.

Des procédés permettant d'obtenir les composés de formule (III) selon ce qui vient d'être décrit sont décrits dans l'ouvrage de Pataï, The chemistry of carboxylic acids and esters, vol. 5, chapitre 9, pages 425-428, édité en 1969 par Interscience/Wiley dans un article de Satchell ainsi que dans Houben-Weyl, Methoden der organischen Chemie, 1952, Vol. VIII, chapitre 5, pages 676-680.

La préparation des 2-(aroylhydrazino) pyridines de formule (III) peut encore s'effectuer commodément par réaction d'un dérivé de pyridine de formule (VIII) avec un arylhydrazide de formule Ar-CO-NH-$NH_2$ dans lesquelles les symboles T, X, Y, Z et Ar ont les significations déjà indiquées pour les formules (I) et (VIII). La réaction se déroule selon le schéma :

$$(VIII) + Ar\text{-}CO\text{-}NH\text{-}NH_2 \rightarrow (III)$$

Cette réaction est avantageusement conduite à température comprise entre 50 et 150 °C, dans des solvants tels que les alcools ou les solvants aromatiques, par exemple la pyridine ou les hydrocarbures tels que le toluène. La proportion de réactifs est avantageusement voisine de la stoechiométrie, par exemple comprise dans un rapport molaire allant de 0,8 à 1,2. La réaction peut être favorisée par la présence d'une quantité, également voisine de la stoechiométrie, d'un agent basique tel qu'un alcoolate ou un bicarbonate alcalin.

Les arylhydrazides de formule Ar-CO-NH-$NH_2$ peuvent être obtenus par réaction d'hydrate d'hydrazine avec un acide ou l'un de ses dérivés tels que les esters, les halogénures ou les anhydrides. La réaction s'effectue avantageusement à température comprise entre 0 et 150°C, de préférence en présence d'un solvant tel qu'un alcool inférieur, la proportion molaire d'hydrate d'hydrazine par rapport à l'autre réactif étant généralement comprise entre 1,01 et 1,5.

Des procédés permettant d'obtenir les arylhydrazides selon ce qui vient d'être décrit sont décrits dans l'ouvrage de Pataï, The chemistry of amides, vol.11, chapitre 10, pages 515-600, édité en 1970 par Interscience/Wiley dans un article de Paulsen et Stoye, ainsi que dans Organic Reactions, the Curtius reaction, 1962, Vol. III, chapitre 9, pages 366-369 édité par Wiley.

Les amidrazones de formule (IV) peuvent être préparées à partir de 2-hydrazinopyridines de formule (V) selon le schéma réactionnel suivant :

$$(V) + Ar\text{-}C(=NH)\text{-}U_3 \rightarrow (IV) + U_3H$$

les divers radicaux de ces réactifs et produits de réaction ayant même signification que dans les formules qui précédent, et $U_3$ représente un radical alkoxy ou alkylthio, de préférence inférieur.

La réaction s'effectue avantageusement à température comprise entre 0 et 30°C, de préférence dans un solvant de type alcool.

Des procédés permettant d'obtenir les amidrazones de formule (IV) selon ce qui vient d'être décrit sont décrits dans l'ouvrage de Pataï, The chemistry of amidines and imidates, vol.20, chapitre 10, pages 491-545, édité en 1975 par Interscience/Wiley dans un article de Watson, ainsi que dans un article de Neilson et al., Chemical Review, 1970, Vol. 70, pages 151-170.

Les iminoéthers de formule Ar-C(=NH)-$U_3$ dans lesquels $U_3$ représente un groupe alkoxy peuvent être préparés par réaction, entre -20 et +30 °C, d'un nitrile aromatique Ar-CN avec un alcool inférieur, de préférence un alcanol, avantageusement dans un solvant de tyge éther, par exemple l'éther éthylique ou le 1,2-diméthoxyéthane ou le dioxanne, ou un hydrocarbure, de préférence aliphatique, halogéné tel que le chloroforme. Plus précisément la réaction s'effectue en milieu anhydre en présence d'HCl (gazeux). Des procédés permettant d'obtenir ces iminoéthers de formule Ar-C(=NH)-$U_3$ sont décrits dans l'ouvrage de Pataï, The chemistry of amidines and imidates, vol.20, chapitre 9, pages 385-489, édité en 1975 par Interscience/Wileg dans un article de Neilson, ainsi que dans un article de Roger et Neilson, Chemical Review, 1961, Vol. 61, pages 179-211.

Les thioimidates de formule Ar-C(=NH)-$U_3$ dans lesquels $U_3$ représente un groupe alkylthio peuvent être préparés par alkylation d'arylthiobenzamides par un procédé tel que décrit par Doyle et al. dans Synthesis, 583 (1974). Ces arylthiobenzamides eux-mêmes peuvent être obtenus par exemple comme indiqué dans l'ouvrage de Pataï, The chemistry of amides, vol.11, chapitre 8, pages 383-475, édité en 1970 par Interscience-/Wiley dans un article de Walta et Vos.

Les 2-hydrazinopyridines de formule (V) peuvent être obtenues par réaction d'hydrate d'hydrazine avec une 2-halogénopyridine de formule (VIII) à température comprise entre 0 et 120°C, en présence ou en absence de solvant. Comme solvant on peut en particulier citer les solvants polaires tels que les alcools, la pyridine, le diméthyl sulfoxyde. La quantité molaire d'hydrate d'hydrazine est généralement comprise entre 3 et 15 fois la quantité de produit de formule (VIII) mis en oeuvre. Des procédés permettant d'obtenir les composés de formule (V) selon ce qui vient d'être décrit sont décrits par Enders dans Houben-Weyl, Methoden der organischen Chemie, 4° édition, 1967, Vol. X-2, chapitre 5, pages 252-287.

Les 2-halogénopyridines de formule (VIII) peuvent en particulier être préparées par un procédé semblable à ceux décrits par Klinsberg dans The chemistry of Heterocyclic compounds, pyridine and its derivatives, parts I - IV, édité en 1974-1975 par Interscience/Wiley , et par Abramovitch, dans The chemistry of Heterocyclic compounds, Pyridine and its derivatives, supplements 1 - 5, édité en 1960-1964 par Interscience/Wiley.

Les exemples suivants, donnés à titre non limitatif, illustrent l'invention et montrent comment elle peut être mise en oeuvre. Dans ces exemples, le symbole Ac désigne le radical $CH_3$-CO-, le symbole DMF désigne le diméthylformamide. Par le mot amidrazone on désigne des composés ayant un groupe

```
          /
-NH-N= C
          \
           NH2
```

Les exemples 1 à 14 illustrent la préparation de composés herbicides selon l'invention. Les exemples I-1 à I-10 illustrent la préparation de composés intermédiaires des produits précédents.

* Exemple 1 :

6,7 g (0,015 mole) de tétraacétate de plomb sont ajoutés, à température ambiante et sous agitation, à 3,5 g (0,015 mole) de phényl (3'-chloro pyrid-2'-yl) hydrazone (préparée comme à l'exemple I-1) dans 300 ml d'acide acétique glacial. L'agitation est poursuivie une heure à 60°C. Le milieu est concentré à sec sous pression réduite. Le résidu est trituré avec 100 ml d'eau, filtré, lavé à l'eau, séché. On obtient 3,1 g (0,0135 mole) de 8-chloro 3-phényl-s-triazolo-[4,3-a] pyridine qui fond a 153°C (rendement 90 %).

* Exemple 2 :

Un mélange de 2,8 g (0,025 mole) de 3-formyl thiophène et 3,1 g (0,025 mole) de 2-hydrazino 3-méthyl pyridine (préparée comme à l'exemple I-8) dans 30 ml d'éthanol contenant quelques gouttes d'HCl concentré,

est porté à l'ébullition pendant deux heures. 70 ml (0,125 mole) d'une solution éthanolique de $FeCl_3,6H_2O$ sont ajoutés progressivement et l'on poursuit le chauffage à ébullition pendant deux heures. Le milieu réactionnel est concentré à sec sous pression réduite, repris avec 100 ml d'eau, neutralisé (pH compris entre 8 et 9) à l'aide de $NH_3$ gazeux, extrait par $CH_2Cl_2$. Après concentration des phases organiques, séchage du résidu et recristallisation à partir d'éthanol aqueux, on obtient 6,05 g (0,02180 mole) de 8-méthyl 3-(thién-3'-yl) -s-triazolo-[4,3-a] pyridine qui fond à 165°C (rendement 87 %).

* Exemple 3 :

A température ambiante, 1,25 ml de brome dans 5 ml d'acide acétique glacial, sont ajoutés à une suspension de 6,15 g (0,075 mole) d'acétate de sodium anhydre dans 50 ml d'acide acétique glacial contenant 6,65 g (0,025 mole) de phényl (3'-trifluorométhyl pyrid-2'-yl) hydrazone préparée comme à l'exemple I-2. Le milieu réactionnel est agité une heure, versé dans 300 ml d'une solution aqueuse de NaOH,2N. Le précipité est filtré, lavé à l'eau, séché. On obtient 4,75 g (0,018 mole) de 3-phényl 8-trifluorométhyl -s-triazolo-[4,3-a] pyridine qui fond à 197°C (rendement 72 %).

* Exemple 4 :

3,15 g (0,025 mole) de 2-formyl 3-méthyl thiophène en solution dans 20 ml d'éthanol sont ajoutés sous agitation et à température ambiante à une solution de 3,6 g (0,025 mole) de 3-chloro 2-hydrazino pyridine (préparée comme à l'exemple I-7) dans 30 ml d'éthanol acidifié avec un peu d'HCl. On chauffe à l'ébullition pendant 2 heures, concentre jusqu'à l'état sec. On obtient de la 3-méthyl thién-2-yl (3'-chloro pyrid-2'yl) hydrazone Celle-ci est dissoute dans 50 ml de nitrobenzène et oxydée à l'air par chauffage pendant 4 heures à l'ébullition. La solution est concentrée sous pression réduite. Le solide recueilli par refroidissement est solubilisé dans 20 ml d'HCl N, puis reprécipité par neutralisation avec une solution ammoniacale. On obtient 4,45 g (0,0178 mole) de 3-(3'-méthyl thién-2'-yl) 8-chloro-s-triazolo-[4,3-a] pyridine qui fond à 135°C (rendement 71 %).

* Exemple 5 :

Un mélange de 1,25 g (0,005 mole) de 3-méthyl 2-(3'-méthyl thièn-2'-oylhydrazino) pyridine (préparée comme à l'exemple I-5) et 7,65 g de $POCl_3$ dans 25 ml de toluène, est porté au reflux pendant deux heures. Le milieu réactionnel est concentré, repris avec 50 ml d'eau glacée, alcalinisé (pH compris entre 8 et 9) à l'aide d'une solution aqueuse de $KHCO_3$, puis extrait avec $CH_2Cl_2$. La solution organique est séchée sur $MgSO_4$ puis concentrée . Le résidu est lavé à l'isopropanol puis séché. On obtient 0,8 g (0,0035 mole) de 8-méthyl 3-(3'-méthyl thién-2'-yl)-s-triazolo-[4,3-a] pyridine qui fond à 94°C (rendement 70 %).

* Exemple 6 :

4,2g (0,0425 mole) de $SOCl_2$ sont ajoutés goutte à goutte à 0°C à une solution de 13,1 g (0,05 mole) de 2-(2'-chloro benzoyl hydrazino) 3-méthyl pyridine (préparée comme à l'exemple I-3) dans 50 ml de pyridine anhydre. Le mélange réactionnel est agité une heure à 0°C puis filtré. L'insoluble est chauffé à l'ébullition pendant 3 heures dans 50 ml d'acétonitrile. La solution est concentrée et le résidu recristallisé. On obtient 5,75 g (0,0235 mole) de 8-méthyl 3-(2'-chloro phényl)-s-triazolo-[4,3-a] pyridine qui fond à 72 °C (rendement 47 %).

* Exemple 7 :

A température ambiante, 3,85 g (0,015 mole) de 2-(2',4'-diméthyl benzoylhydrazino) 3-méthyl pyridine (préparée comme à l'exemple I-4) sont dissous dans 30 ml d'acide acétique glacial. Le mélange réactionnel est porté à l'ébullition pendant 12 heures puis concentré à sec. Le résidu est lavé à l'eau puis au pentane, recristallisé dans l'éthanol absolu. On obtient 1,65 g (0,007 mole) de 8-méthyl 3-(2',4'-diméthyl phényl)-s-triazolo-[4,3-a] pyridine qui fond à 106°C (rendement 47 %).

* Exemple 8 :

6,8 g (0,03 mole) de $N^1$- (5'-méthyl pyrid-2'-yl) phényl amidrazone (préparée comme à l'exemple I-6) sont dissous dans 25 ml d'acide formique. L'ensemble est porté une heure au reflux, refroidi, versé dans 200 ml d'eau. Le précipité est récupéré par filtration, lavé à l'eau, séché. On obtient 4,7 g (0,0225 mole) de 3-phényl 6-méthyl-s-triazolo-[4,3-a] pyridine qui fond à 160°C (rendement 75 %).

* Exemple 9 :

Un mélange de 3,1 g (0,025 mole) de 2-hydrazino 3-méthyl pyridine (préparée comme à l'exemple I-8) et 3,5 g (0,025 mole) d'acide 4-fluoro benzoïque est chauffé 3 heures à 180°C puis refroidi, puis mélangé à 80 ml de chloroforme bouillant. La solution est filtrée, concentrée à sec. Le résidu est traité avec une solution aqueuse de $Na_2CO_3$, filtré, lavé à l'eau, séché, recristallisé dans le méthanol. On obtient 2,3 g (0,01 mole) de 3-(4'-fluoro phényl) 8-méthyl -s-triazolo-[4,3-a] pyridine qui fond à 166°C (rendement 40 %).

* Exemple 10 :

Un mélange de 3,1 g (0,025 mole) de 2-hydrazino 3-méthyl pyridine (préparée comme à l'exemple I-8) et 3,1 g (0,025 mole) d'acide picolinique est chauffé 4 heures à 180°C, refroidi, dilué à l'aide de 100 ml d'éthanol aqueux, alcalinisé (pH égal à environ 8-9) avec une solution de NaOH diluée. Le précipité est récupéré par filtration, lavé à l'eau, séché, recristallisé à partir d'éthanol aqueux. On obtient 1,0 g (0,00475 mole) de 8-méthyl 3-(pyrid-2'-yl) -s-triazolo-[4,3-a] pyridine qui fond à 141°C (rendement 19 %).

* Exemple 11 :

On reproduit l'exemple 9 mais en remplaçant l'acide 4-fluoro benzoïque par de l'acide 2-fluoro benzoïque. On obtient 4,1 g (0,018 mole) de 3-(2'-fluoro phényl) 8-méthyl -s-triazolo-[4,3-a] pyridine qui fond à 130°C (rendement 72 %).

* Exemple 12 :

A température ambiante, 5,05 g (0,05 mole) de triéthylamine et 3,1 g (0,025 mole) de 2-hydrazino 3-méthyl pyridine (préparée comme à l'exemple I-8) sont ajoutés successivement à une suspension de 5 g (0,025 mole) de chlorhydrate de 4-méthyl benzimidate d'éthyle dans 50 ml d'éthanol. Le milieu réactionnel est porté à l'ébullition pendant une heure, puis est concentré, refroidi, versé dans 250 ml d'eau froide. Le mélange est extrait à l'aide de $HCCl_3$. La solution organique est séchée, concentrée à sec. On obtient 3 g (0,0135 mole) de 3-(4'-méthyl phényl) 8-méthyl -s-triazolo-[4,3-a] pyridine qui fond à 145°C (rendement 54 %).

* Exemple 13 :

On reproduit l'exemple 2 mais en remplaçant les 2,8 g de 3-formyl thiophène par 4,6 g (0,025 mole) de 4-phénylbenzaldéhyde. On obtient 2,3 g (0,008 mole) de 8-méthyl 3-(4'-phényl phényl) -s-triazolo-[4,3-a] pyridine qui fond à 156°C (rendement 32 %).

* Exemple 14 :

On reproduit l'exemple 12 en remplaçant le chlorhydrate de 4-méthyl benzimidate d'éthyle par du chlorhydrate de benzimidate de méthyle. On obtient 3,5 g (0,0168 mole) de 3-phényl 8-méthyl -s-triazolo[4,3-a] pyridine qui fond à 118°C (rendement 67 %).

* Exemple 15 :

On reproduit l'exemple 4 mais en remplaçant :
1) les 3,15 g de 2-formyl 3-méthyl thiophène par 4,00 g (0,025 mole) de 4- méthylthio benzaldéhyde, et
2) 3,6 g de 3-chloro 2-hydrazino pyridine par 3,1 g (0,025 mole) de 2-hydrazino 3-méthyl pyridine. On obtient de la 4-méthylthio phényl (3'-méthyl pyrid-2'yl) hydrazone qui est dissoute dans 50 ml de nitro-benzène, puis traitée comme à l'exemple 4. On obtient 4,60 g (0,018 mole) de 3-(4'-méthylthio phényl) 8-méthyl - s - triazolo - [4,3-a] pyridine qui fond à 137°C (rendement : 72 %).

* Exemple 16

2,0 g (0,018 mole) de 2-formyl thiazole en solution dans 20 ml d'éthanol sont ajoutés sous agitation et à température ambiante à une solution de 2,4 g (0,018 mole) de 3-chloro 2-hydrazino pyridine (préparée comme à l'exemple I-7) dans 30 ml d'éthanol contenant 0,2 g d'acide p-toluène sulfonique. On chauffe à l'ébullition pendant 2 heures, concentre jusqu'à l'état sec. On obtient de la thiazol-2-yl (3'-chloro pyrid-2'yl) hydrazone.

Celle-ci est dissoute dans 50 ml d'éthanol. A température ambiante, 5,1 g (0,018 mole) de chloramine T sont ajoutés rapidement à la solution éthanolique précédente. Le milieu réactionnel est agité pendant 30 minutes. Après évaporation du solvant, le résidu est chromatographié (éluant heptane/acétate d'éthyle 40/60). On obtient 1,50 g (0,0063 mole) de 3-(thiazol-2'-yl) 8-chloro-s-triazolo-[4,3-a] pyridine qui fond à 218°C (rendement 36 %).

*Exemple 17

On reproduit l'exemple 16 mais en remplaçant la 3-chloro 2-hydrazino pyridine par de la 2-hydrazino 3-méthyl pyridine (préparée comme à l'exemple I-8). On obtient 1,26 g (0,0058 mole) de 3-(thiazol-2'yl) 8-méthyl-s-triazolo-[4,3-a] pyridine qui fond à 140°C (rendement 32 %).

*Exemple 18

On reproduit l'exemple 17 mais en remplaçant le 2-formyl thiazole par du phénoxy-4 benzaldéhyde. On obtient 4,7 g (0,0156 mole) de 3-(4'-phénoxy phényl) 8-méthyl-s-triazolo-[4,3-a] pyridine qui fond à 132°C (rendement 87 %).

*Exemple 19

On reproduit l'exemple 16 mais en remplaçant le 2-formyl thiazole par du phénoxy-4 benzaldéhyde. On obtient 5,0 g (0,0155 mole) de 3-(4'-phénoxy phényl) 8-chloro-s-triazolo-[4,3-a] pyridine qui fond à 145°C (rendement 86 %).

*Exemple 20

On reproduit l'exemple 5 mais en remplaçant la 3-méthyl 2-(3'-méthyl thièn-2'-oylhydrazino) pyridine par de la 3-méthyl 2-(4'-méthyl 1',2',3'-thiadiazol-5'-oylhydrazino) pyridine. On obtient 0,3 g (0,0013 mole) de 3-(4'-méthyl 1',2',3'-thiadiazol-5'-yl) 8-méthyl-s-triazolo-[4,3-a] pyridine qui fond à 180°C (rendement 26 %).

On reproduit l'un des exemples 4 ou 5 pour obtenir les composés des exemples suivants :

*Exemple 21

8-bromo, 3-[3'-méthyl thién-2'yl]-s triazolo-[4,3-a] pyridine,

*Exemple 22

8-trifluorométhyl, 3-[3'-méthyl thién-2'yl]-s-triazolo [4,3-a] pyridine,

*Exemple 23

8-méthyl, 3-[2'-méthyl thién-2'yl]-s triazolo-[4,3-a] pyridine,

*Exemple 24

8-bromo, 3-phényl-s triazolo [4,3-a] pyridine,

*Exemple 25

8-chloro, 3-[2'-méthyl thién-3'yl]-s triazolo [4,3-a] pyridine,

*Exemple 26

8-chloro, 3-[thién-3'yl]-s triazolo [4,3-a] pyridine,

*Exemple 27

8-méthyl, 3-[1'-méthyl pyrrol-2'yl]-s triazolo [4,3-a] pyridine,

*Exemple 28

8-chloro, 3-[3'5'-diméthyl thién-2'yl]-s triazolo [4,3-a] pyridine,

*Exemple 29

7,8-Diméthyl,3-phényl-s triazolo [4,3-a] pyridine,

*Exemple 30

8-méthyl, 3-[3'5'-diméthyl thién-2'yl]-s triazolo [4,3-a] pyridine,

*Exemple 31

8-éthyl, 3-[3'-méthyl thién-2'yl]-s triazolo [4,3-a] pyridine,

*Exemple 32

8-éthyl, 3-phényl-s triazolo [4,3-a] pyridine,

*Exemple 33

8-éthyl, 3-[1'-méthyl pyrrol-2'yl]-s-triazolo [4,3-a] pyridine,

*Exemple 34

8-éthyl, 3-[thién-3'yl]-s-triazolo [4,3-a] pyridine,

*Exemple 35

8-trifluorométhyl, 3-[1'-méthyl pyrrol-2'yl]-s-triazolo [4,3-a] pyridine,

*Exemple 36

8-trifluorométhyl, 3-[pyrrol-2'yl]-s-triazolo [4,3-a] pyridine,

*Exemple 37

8-chloro, 3-[4'-méthyl thién-2'yl]-s-triazolo [4,3-a] pyridine,

*Exemple 38

7,8-Diméthyl,3-[3'-méthyl thién-2'yl]-s-triazolo [4,3-a] pyridine,

*Exemple 39

8-méthyl, 3-[4'-méthyl thién-2'yl]-s-triazolo [4,3-a] pyridine,

*Exemple 40

8-trifluorométhyl, 3-[thièn -3'yl]-s-triazolo [4,3-a] pyridine,

*Exemple 41

8-méthyl, 3-[4'-isopropyl phényl]-s-triazolo [4,3-a] pyridine,

*Exemple 42

8-méthyl, 3-[4'-bromo thièn-2'yl]-s-triazolo [4,3-a] pyridine,

*Exemple 43

8-chloro, 3-[4'-bromo thièn-2'yl]-s-triazolo [4,3-a] pyridine,

*Exemple 44

8-méthyl, 3-[3'-chloro thièn-2'yl]-s-triazolo-[4,3-a] pyridine.

*Exemple 45

8-chloro, 3-[3'-chloro thièn-2'yl]-s-triazolo-[4,3-a] pyridine.

*Exemple 46

8-méthyl, 3-[3'-bromo thièn-2'yl]-s-triazolo-[4,3-a] pyridine.

*Exemple 47

8-chloro, 3-[3' bromo thièn-2'yl]-s-triazolo-[4,3-a] pyridine.
Les produits ci-dessus sont obtenus selon les méthodes décrites aux exemples 4 et 5.

* Exemple I-1 :

Sous agitation et à température ambiante, 5,35 g (0,05 mole) de benzaldéhyde dans 20 ml d'éthanol sont ajoutés à une solution de 7,2 g (0,05 mole) de 3-chloro 2-hydrazino pyridine dans 30 ml d'éthanol contenant quelques gouttes d'HCl concentré. L'ensemble est porté au reflux pendant deux heures puis concentré. Après refroidissement, le résidu solide est récupéré par filtration, séché, recristallisé dans l'éthanol. On obtient 10,45 g (0,045 mole) de phényl (3'-chloro pyrid-2'-yl) hydrazone qui fond à 151 °C (rendement 91 %).

* Exemple I-2 :

5,35 g (0,05 mole) de benzaldéhyde dans 20 ml d'éthanol, sont ajoutés à une solution de 13,25 g (0,05 mole) de 3-trifluorométhyl 2-hydrazino pyridine (préparée comme à l'exemple I-9) dans 30 ml d'éthanol contenant 0,3 ml d'acide acétique. Le mélange est porté au reflux pendant 2 heures, concentré, refroidi, dilué avec 5 ml d'eau. Le précipité est récupéré par filtration, séché. On obtient 11,15 g (0,042 mole) de phényl (3'-trifluorométhyl pyrid-2'-yl) hydrazone qui fond à 119°C (rendement 84 %).

* Exemple I-3 :

8,75 g (0,05 mole) de chlorure de 2-chloro benzoyle sont additionnés lentement et à la température du bain d'eau glacée, à 6,15 g (0,05 mole) de 3-méthyl 2- hydrazino pyridine (préparée comme à l'exemple I-8) dans 50 ml de pyridine anhydre. Le mélange réactionnel est agité 4 heures à température ambiante, puis versé dans 100 ml d'eau glacée. Après séjour de 12 h à 0°C, le mélange est filtré, le précipité lavé à l'eau, séché et recristallisé à partir d'éthanol aqueux. On obtient 10,45 g (0,04 mole) de 2-(2'-chloro benzoylhydrazino) 3-méthyl pyridine qui fond à 115 °C (rendement 80 %).

* Exemple I-4 :

8,45 g (0,05 mole) de chlorure de 2,4-diméthyl benzoyle sont ajoutés lentement à 0°C à 6,15 g (0,05 mole) de 3-méthyl 2-hydrazino pyridine (préparée comme à l'exemple I-8) dans 200 ml de $CH_2Cl_2$ contenant 5,5 g (0,055 mole) de triéthylamine. Le mélange réactionnel est porté au reflux pendant 3 heures, refroidi, filtré, concentré. Le résidu est lavé à l'eau, séché, recristallisé à partir d'éthanol aqueux. On obtient 9,45 g (0,037 mole) de 2-(2',4'-diméthyl benzoylhydrazino) 3-méthyl pyridine qui fond à 120°C (rendement 74 %).

* Exemple I-5 :

On chauffe 8 heures à l'ébullition, dans 30 ml de n-butanol, un mélange de 3,9 g (0,025 mole) de 3-méthyl thién-2-yl carboxylate de méthyle et 3,1 g (0,025 mole) de 2-hydrazino 3-méthyl pyridine (préparée comme à l'exemple I-8). On concentre à sec, recristallise dans le toluène. On obtient 5,7 g (0,023 mole) de 3-méthyl 2-(3'-méthyl thièn-2'-oylhydrazino) pyridine fondant à 196°C (rendement 92 %).

* Exemple I-6 :

A température ambiante, 12 g (0,07 mole) de chlorhydrate de benzimidate de méthyle sont additionnés à une solution de 8,65 g (0,07 mole) de 2-hydrazino 5-méthyl pyridine (préparée comme à l'exemple I-10) dans un mélange de 150 ml d'éthanol absolu et de 7,1 g (0,07 mole) de triéthylamine. L'ensemble est agité 24 heures à température ambiante. Le précipité est récupéré par filtration, lavé avec de l'éthanol puis séché. On obtient 9,95 g (0,044 mole) de $N^1$-(5'-méthyl pyrid-2'-yl) phényl amidrazone qui fond à une température voisine de 166°C (rendement 63 %).

* Exemple I-7 :

125 ml d'hydrazine à 85 % sont ajoutés lentement et à température ambiante, à une solution de 74 g (0,5 mole) de 2,3-dichloro pyridine dans 400 ml d'éthanol. Le mélange réactionnel est porté à l'ébullition pendant 24 heures puis refroidi à 0°C. Le solide précipité est récupéré par filtration, recristallisé à partir d'éthanol. On obtient 66,05 g (0,46 mole) de 2-hydrazino 3-chloro pyridine qui fond à 167°C (rendement 92 %).

* Exemple I-8 :

Un mélange de 51,6 g (0,3 mole) de 2-bromo 3-méthyl pyridine et 110 ml d'hydrazine à 85 % est porté à l'ébullition pendant 20 heures. Après retour à température ambiante, le précipité est récupéré par filtration, lavé avec de l'éther d'isopropyle, solubilisé dans du $CH_2Cl_2$. La solution organique est filtrée, concentrée à sec. On obtient 26,45 g (0,215 mole) de 2-hydrazino 3-méthyl pyridine qui fond à 122°C (rendement 71 %).

* Exemple I-9 :

On reproduit l'exemple I-8 mais en remplaçant les 51,6 g de 2-bromo 3-méthyl pyridine par 54,45 g de 2-chloro 3-trifluorométhyl pyridine et on chauffe pendant 24 h. On obtient 34,55 g (0,195 mole) de 2-hydrazino 3-trifluorométhyl pyridine fondant à 63°C (rendement 65 %).

* Exemple I-10 :

On reproduit l'exemple I-8 mais en remplaçant la 2-bromo 3-méthyl pyridine par une même quantité de 2-bromo 5-méthyl pyridine. On obtient 21,55 g (0,175 mole) de 2-hydrazino 5-méthyl pyridine fondant à 73°C (rendement 58 %).

Les exemples ci-dessous, donnés à titre non limitatif, illustrent l'utilisation des produits selon l'invention et leur application au désherbage.

Dans ces exemples, on a utilisé les abréviations suivantes :

| Abréviations | Nom français Mauvaises herbes | Nom latin |
|---|---|---|
| AVE | Folle avoine | Avena fatua |
| ECH | Panisse | Echinochloa crus-galli |
| CHR | Chrysanthème des moissons | Chrysanthemum segetum |
| POR | Pourpier | Portulaca oleracea |
| DIG | Digitaire | Digitaria sanguinalis |
| SIN | Moutarde | Sinapis alba |
| ALO | Vulpin | Alopecurus myosuroides |
| IPO | Ipomée | Ipomea purpurea |
| ABU | Abutilon | Abutilon theophrasti |
| SOL | Morelle noire | Solanum nigrum |
| STE | Stellaire | Stellaria media |
| CEN | Bleuet | Centaurea cyanus (vérifier) |
| SES | Sesbania cultures | Sesbania exaltata |
| SET | Setaire | Setaria faberii |
| CHY | Chrysanthème | Chrysanthemum segetum |

| Abréviations | Nom français | Nom latin Cultures |
|---|---|---|
| TRZ | Blé | Triticum aestivum |
| ZEA | Maïs | Zea Mays |
| ORY | Riz | Oryza sativa |
| GLX | Soja | Glycine maximum |
| GOS | Coton | Gossypium hirsutum |
| HEL | Tournesol | Helianthus annuus |
| BRS | Colza | Brassica napus |

Exemple $U_1$ :

Application herbicide, en prélevée des espèces végétales.

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 % en poids de Cemulsol NP 10 (agent tensioactif) constitué d'alkylphénol polyéthoxylé, notamment de nonylphénol polyéthoxylé) et 0,04 % en poids de tween 20 (agent tensio-actif constitué d'un oléate de dérivé polyoxyethyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 60 % d'humidité relative.

Au bout de 24 jours, on mesure un pourcentage (D) de destruction du nombre de plantes dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire détruit et réduit par le produit est donc donné par la formule

$$D + \frac{RT\ (100 - D)}{100} = A$$

Cette valeur A est transformée en notation de 0 à 5 selon l'échelle suivante :

<u>notation</u>

| | | |
|---|---|---|
| 0 < A < 10 | 0 | pas d'effet |
| 10 < A < 30 | 1 | |
| 30 < A < 50 | 2 | |
| 50 < A < 70 | 3 | |
| 70 < A < 90 | 4 | |
| 90 < A < 100 | 5 | destruction complète. |

Les résultats obtenus sont présentés ci-après :

| composé selon exemple n°(appliqués à la dose de 4 kg/ha) | 6 | 8 | 9 | 7 | 4 | 2 | 10 | 3 | 5 | 1 |
|---|---|---|---|---|---|---|---|---|---|---|
| activité A en prélevée | | | | | | | | | | |
| AVE | 0 | 0 | 2 | 0 | 5 | 5 | 1 | 4 | 3 | 5 |
| ECH | 3 | 0 | 2 | 1 | 5 | 4 | 3 | 5 | 5 | 5 |
| ALO | 2 | 1 | 4 | 3 | 5 | 5 | 4 | 5 | 5 | 5 |
| DIG | 5 | 5 | 5 | 3 | 5 | 5 | 5 | 5 | 5 | 5 |
| IPO | 4 | 3 | 4 | 5 | 5 | 3 | 1 | 5 | 2 | 5 |
| SIN | 5 | 5 | 5 | 3 | 5 | 5 | 2 | 5 | 5 | 5 |
| ABU | 4 | 5 | 5 | 5 | 5 | 4 | 3 | 5 | 5 | 5 |
| SOL | 5 | 5 | 0 | 5 | 5 | 5 | 5 | 5 | 5 | 5 |

ACTIVITE DE PRELEVEE

| EXEMPLES | ECH | ALO | DIG | SIN | ABU | SOL | STE |
|---|---|---|---|---|---|---|---|
| 16 | 5 | 5 | 5 | 5 | 5 | 5 | 5 |
| 17 | 4 | | 5 | 5 | 2 | 5 | 5 |
| 20 | 4 | 5 | 5 | 5 | 5 | 5 | 5 |
| 22 | 4 | | 4 | 5 | 5 | 1 | 0 |
| 23 | 3 | | 4 | 5 | 1 | 5 | 5 |
| 24 | 4 | 4 | 4 | 2 | 0 | 5 | 5 |
| 25 | 3 | 5 | 5 | 1 | 0 | 5 | 5 |
| 27 | 4 | | 5 | 5 | 2 | 4 | 4 |
| 31 | 5 | 5 | 5 | 5 | 5 | 5 | 5 |
| 32 | 5 | 5 | 5 | 5 | 5 | 5 | 5 |
| 33 | 5 | 5 | 5 | 5 | 5 | 5 | 5 |
| 34 | | 5 | 5 | 5 | 5 | 5 | |
| 35 | 5 | 5 | 5 | 5 | 5 | 5 | 5 |
| 37 | 5 | 5 | 5 | 5 | 0 | 5 | |
| 38 | 5 | 4 | 5 | 0 | 3 | 5 | 5 |
| 39 | 3 | 5 | 5 | 1 | 0 | 5 | 5 |
| 40 | 5 | 5 | 4 | 0 | 3 | 5 | 5 |
| 41 | 4 | 4 | 5 | 2 | 1 | 5 | 4 |
| 42 | 3 | 5 | 5 | 5 | 0 | 5 | 5 |
| 43 | 5 | 5 | 5 | 5 | 5 | 5 | |

| | | ACTIVITES DE PRELEVEE CULTURES | | | | | | |
|---|---|---|---|---|---|---|---|---|
| EXEMPLES | DOSE Kg/ha | BRS | GOS | GLX | HEL | TRZ | ZEA | ORY |
| 17 | 2 | 0 | 0 | 1 | 0 | 0 | 0 | 0 |
| 27 | 2 | 5 | | 0 | 0 | 1 | 0 | 0 |
| 31 | 1 | 0 | 0 | 0 | 0 | 0 | 0 | 0 |
| 36 | 2 | 5 | 0 | 0 | 4 | 1 | 0 | 1 |
| 38 | 1 | 5 | 0 | 0 | 0 | 0 | 0 | 0 |

| | | ACTIVITES DE PRELEVEE MAUVAISES HERBES | | | | |
|---|---|---|---|---|---|---|
| EXEMPLES | DOSE Kg/ha | STE | CHY | SOL | DIG | SET |
| 17 | 2 | 5 | 5 | 5 | 5 | 5 |
| 27 | 2 | 5 | 5 | 1 | 5 | 5 |
| 31 | 1 | 2 | | 5 | 4 | 4 |
| 36 | 2 | 5 | 5 | 5 | 5 | 5 |
| 38 | 1 | 5 | 5 | 5 | 5 | 5 |

| | | ACTIVITES DE PRELEVEE | | | | | | |
|---|---|---|---|---|---|---|---|---|
| | | CULTURES | | | MAUVAISES HERBES | | | |
| EXEMPLES | DOSE Kg/ha | GLX | TRZ | ZEA | STE | ABU | SOL | DIG |
| 32 | 2 | 0 | 0 | 0 | 0 | 5 | 4 | 4 |
| 39 | 1 | 0 | 0 | 0 | 5 | 5 | 5 | 5 |
| 35 | 1 | 0 | 2 | 0 | 4 | 5 | 5 | 5 |
| 33 | 2 | 0 | 0 | 0 | 4 | 1 | 4 | 5 |
| 16 | 2 | 0 | 2 | 0 | 5 | 4 | 5 | 5 |
| 43 | 4 | 1 | 0 | 1 | 5 | 5 | 5 | 5 |

| Composé selon l'exemple n° | 5 | 1 | 12 |
|---|---|---|---|
| Appliqué à la dose de (kg/ha) | 2 | 1 | 4 |
| Activité A en prélevée | | | |
| DIG | 5 | 5 | 4 |
| ECH | 5 | 4 | 1 |
| SET | 4 | 4 | 3 |
| STE | 3 | 4 | 4 |
| SOL | 4 | 5 | 5 |
| CEN | 5 | 4 | 3 |
| GOS | 0 | . | . |
| HEL | 0 | 3 | 2 |
| GLX | 1 | 0 | 1 |
| TRZ | 2 | 3 | 0 |
| ZEA | 0 | 2 | 1 |
| ORY | 1 | 0 | 0 |

Exemples suivants :

Ces exemples montrent que, en prélevée, les produits selon l'invention ont une bonne activité générale sur les mauvaises herbes et une bonne sélectivité sur une ou plusieurs cultures telles que le blé, le maïs, le riz, le coton, le tournesol, le soja, le colza.

Exemple $U_2$ :

Application herbicide, en postlevée des espèces végétales

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation". Le stade de traitement pour les dicotylédones est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouillie utilisée pour le traitement est préparée comme à l'exemple $U_1$.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 60 % d'humidité relative.

La mesure de A (pourcentage de volume foliaire détruit) est effectuée comme à l'exemple $U_1$.

Les résultats obtenus sont présentés ci-après :

| composé selon exemple n° (appliqué à la dose de 4 kg/ha) | 6 | 15 | 9 | 7 | 4 | 2 | 10 | 3 | 5 | 1 | 11 | 13 |
|---|---|---|---|---|---|---|---|---|---|---|---|---|
| activité en post-levée | | | | | | | | | | | | |
| AVE | 2 | 3 | 1 | 2 | 5 | 5 | 4 | 2 | 5 | 2 | 3 | 2 |
| ECH | 3 | 4 | 5 | 4 | 5 | 5 | 4 | 3 | 5 | 5 | 5 | 5 |
| ALO | 2 | 4 | 4 | 5 | 5 | 5 | 2 | 4 | 5 | 5 | 4 | . |
| DIG | 2 | 3 | 3 | 3 | 5 | 4 | 4 | 3 | 5 | 4 | 4 | 5 |
| IPO | 2 | 4 | 4 | 5 | 5 | 5 | 4 | 3 | 5 | 5 | 5 | 5 |
| SIN | 5 | 5 | 5 | 5 | 5 | 5 | 5 | 5 | 5 | 5 | 5 | 5 |
| ABU | 3 | 4 | 2 | 3 | 5 | 5 | 4 | 0 | 5 | 5 | 3 | 5 |
| SOL | 4 | 4 | 5 | 5 | 5 | 5 | 5 | 5 | 5 | 5 | 3 | 5 |

| EXEMPLES | ACTIVITES DE POSTLEVEE | | | | | | |
|---|---|---|---|---|---|---|---|
| | ECH | ALO | DIG | SIN | ABU | SOL | STE |
| 16 | 4 | 5 | 4 | 5 | 3 | 5 | 5 |
| 17 | 5 | 1 | 2 | 5 | 3 | 5 | 5 |
| 18 | 3 | 5 | 5 | 5 | | | |
| 20 | 2 | 4 | 2 | 4 | 2 | 2 | 5 |
| 22 | 4 | 2 | 3 | 5 | 0 | 4 | 3 |
| 24 | 2 | 3 | 2 | 5 | 1 | 2 | 4 |
| 25 | 4 | 5 | 4 | 1 | 0 | 2 | 5 |
| 26 | 4 | 4 | 4 | 5 | 5 | 4 | 5 |
| 28 | 3 | 3 | 4 | 5 | 4 | 5 | 5 |
| 29 | 5 | 2 | 4 | 5 | 5 | 4 | 4 |
| 31 | 5 | 5 | 5 | 5 | 5 | 5 | 5 |
| 32 | 5 | 5 | 4 | 5 | 4 | 5 | 5 |
| 33 | 5 | 5 | 5 | 5 | 5 | 5 | 5 |
| 34 | | 5 | 5 | 5 | 5 | 5 | |
| 35 | 5 | 5 | 5 | 5 | 5 | 4 | 5 |
| 37 | | 5 | 4 | 5 | 5 | 5 | |
| 38 | 5 | 3 | 4 | 2 | 5 | 5 | 5 |
| 39 | 5 | 5 | 5 | 5 | 3 | 4 | 5 |
| 40 | 5 | 4 | 5 | 5 | 5 | 5 | 5 |
| 41 | 5 | 5 | 5 | 5 | 5 | 5 | 5 |
| 42 | 4 | 3 | 4 | 5 | 5 | 5 | 5 |
| 43 | 5 | 5 | 1 | 5 | 5 | 5 | |

| composé selon exemple n° | 5 | 1 | 2 | 7 | 10 | 12 | 9 |
|---|---|---|---|---|---|---|---|
| appliqué à la dose de kg/ha) | 0,5 | 2 | 1 | 2 | 1 | 1 | 2 |
| Activité A en postlevée | | | | | | | |
| mauvaises herbes | | | | | | | |
| IPO | 3 | 4 | 3 | 4 | 3 | 4 | 3 |
| SOL | 5 | 5 | 5 | 5 | 5 | 5 | 5 |
| SES | 4 | 5 | 5 | 5 | 2 | 5 | 5 |
| ECH | 5 | 4 | 3 | 4 | 4 | 4 | 3 |
| POR | 5 | 5 | 5 | 4 | 2 | 3 | 4 |
| STE | 2 | 5 | 5 | 4 | 5 | 5 | 5 |
| CHY | 5 | 5 | 5 | 5 | 4 | 5 | 5 |
| cultures | | | | | | | |
| TRZ | 0 | 2 | 0 | 1 | 0 | 0 | 2 |
| ZEA | 0 | 1 | 1 | 0 | 0 | 0 | 0 |
| ORY | 1 | 1 | 1 | 1 | 1 | 0 | 1 |

| | | ACTIVITES DE POSTLEVEE MAUVAISES HERBES | | | | |
|---|---|---|---|---|---|---|
| EXEMPLES | DOSE Kg/ha | GOS | TRZ | ZEA | ORY | GLX |
| 38 | 1 | 1 | 2 | 0 | 0 | |
| 31 | 0,5 | 0 | 1 | 1 | 0 | |
| 37 | 2 | 0 | 0 | 0 | 0 | |
| 36 | 1 | | 1 | 0 | 0 | |
| 28 | 2 | | 0 | 0 | 0 | |
| 42 | 2 | | 1 | 0 | 1 | |
| 41 | 2 | | 1 | 0 | 1 | |
| 32 | 1 | | 1 | 0 | 1 | |
| 29 | 4 | | 0 | 0 | | 1 |
| 16 | 2 | | 0 | 0 | | 1 |
| 21 | 2 | | 0 | 0 | | 0 |
| 17 | 2 | | 1 | 0 | | 0 |
| 39 | 2 | | 1 | 0 | | |
| 25 | 2 | | 0 | 0 | | |
| 26 | 2 | | 0 | 0 | | |
| 35 | 0,5 | | 0 | 0 | | |
| 33 | 1 | | 3 | 0 | | |
| 18 | 1 | | 0 | 0 | | |

| | | ACTIVITES DE POSTLEVEE MAUVAISES HERBES | | | | |
|---|---|---|---|---|---|---|
| EXEMPLES | DOSE Kg/ha | STE | CHY | SES | CEN | SET |
| 38 | 1 | 4 | 5 | 4 | 4 | 4 |
| 31 | 0,5 | 5 | 5 | 3 | 5 | 3 |

| EXEMPLES | DOSE Kg/ha | ACTIVITES DE POSTLEVEE MAUVAISES HERBES | | | | | |
|---|---|---|---|---|---|---|---|
| | | ABU | IPO | CHY | SOL | POL | STE |
| 37 | 2 | 3 | 4 | 5 | 4 | | |
| 36 | 1 | | | 5 | | 3 | 5 |
| 28 | 2 | | | 5 | | 3 | 4 |
| 42 | 2 | | | 5 | | 4 | 4 |
| 41 | 2 | | | 5 | | 4 | 5 |
| 32 | 1 | | | 5 | | 5 | 4 |

| | | ACTIVITES DE POSTLEVEE MAUVAISES HERBES | | | |
|---|---|---|---|---|---|
| EXEMPLES | DOSE Kg/ha | STE | ABU | SOL | SET |
| 29 | 4 | 4 | 5 | 4 | 4 |
| 16 | 2 | 5 | 3 | 5 | 4 |
| 21 | 2 | 5 | 3 | 4 | 4 |
| 17 | 2 | 5 | 5 | 3 | 5 |

| | | ACTIVITES DE POSTLEVEE MAUVAISES HERBES | | | | | | |
|---|---|---|---|---|---|---|---|---|
| EXEMPLES | DOSE Kg/ha | STE | ABU | IPO | SOL | ECH | DIG | SET |
| 39 | 2 | 5 | 5 | 4 | 5 | 5 | 5 | 5 |
| 25 | 2 | 5 | 4 | 5 | 5 | 5 | 5 | 5 |
| 26 | 2 | 4 | 5 | 4 | 4 | 5 | 4 | 4 |
| 35 | 0,5 | 5 | 5 | 4 | 5 | 5 | 3 | 5 |
| 33 | 1 | 5 | 5 | 5 | 5 | 5 | 3 | 5 |
| 28 | 1 | 5 | 5 | 5 | 5 | 1 | 5 | 4 |

En outre, à la dose de 2 kg/ha, le composé de l'exemple 14 a présenté les activités A suivantes :

| IPO | SOL | SES | STE | CHY | GLX | HEL | TRZ | ZEA | ORY |
|---|---|---|---|---|---|---|---|---|---|
| 4 | 5 | 3 | 4 | 5 | 0 | 1 | 0 | 0 | 0 |

Ces exemples montrent que, en postlevée, les composés selon l'invention ont une bonne activité générale sur les mauvaises herbes et une bonne sélectivité sur une ou plusieurs cultures, telles que le blé, le maïs, le riz, le tournesol, le coton, le soja.

Les essais réalisés montrent donc les propriétés avantageuses des composés selon l'invention en tant qu'herbicides, notamment herbicides à large spectre d'activité et pouvant être actifs en prélevée et postlevée.

Pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en mélange avec au moins un support solide ou liquide, acceptable en agriculture et, éventuellement au moins un agent de surface également acceptable en agriculture. Par agent de surface, dans le présent exposé, on entend un agent connu en langue anglaise sous l'appellation "surfactant", ce qui recouvre principalement les agents tensioactifs, mouillants ou dispersants.

Ces compositions font également partie de l'invention. Elles peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc. Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée.

Le support peut être de tout type usuel. Il peut en particulier être solide (argiles, silicates naturels ou synthétiques, silice, résines, cires, engrais solides, etc...) ou liquide (eau ; alcools, notamment le butanol etc..).

L'agent de surface ou l'agent tensioactif peut lui aussi être de tout type usuel. Il peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents.

La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 0,1 % et 50 % en poids.

En ce qui concerne les compositions adaptées au stockage et au transport, elles contiennent plus avantageusement de 0,5 à 95 % (en poids) de substance active, les compositions telles qu'appliquées sur les plantes étant en général nettement plus diluées que les compositions adaptées au stockage et au transport qui sont plus concentrées.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) ; les poudres mouillables ; les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc.

Pour obtenir les poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple F 1 :

* matière active (composé n° 1) 50 %
* alcool gras éthoxylé (agent mouillant) 2,5%
* phényléthylphénol éthoxylé (agent dispersant) 5 %
* craie (support inerte) 42,5%

Exemple F 2 :

* matière active (composé n° 1) 10%
* alcool synthétique oxo de type ramifié, en $C_{13}$ éthoxylé par 8 à 10 oxyde d'éthylène (agent mouillant) 0,75%
* lignosulfonate de calcium neutre (agent dispersant) 12 %
* carbonate de calcium (charge inerte) q.s.p. 100 %

Exemple F 3 : cette poudre mouillable contient les mêmes ingrédients que dans l'exemple précédent, dans les proportions ci-après :

* matière active 75 %
* agent mouillant 1,50%
* agent dispersant 8 %
* carbonate de calcium (charge inerte) q.s.p. 100 %

Exemple F 4 :

* matière active (composé n° 1) 90 %
* alcool gras éthoxylé (agent mouillant) 4 %
* phényléthylphénol éthoxylé (agent dispersant) 6 %

Exemple F 5 :

* matière active (composé n° 1) 50 %
* mélange de tensio-actifs anioniques et non ioniques (agent mouillant) 2,5 %
* lignosulfonate de sodium (agent dispersant) 5 %
* argile kaolinique (support inerte) 42,5 %

Les composés selon l'invention peuvent être formulés sous la forme de granulés dispersables dans l'eau également compris dans le cadre de l'invention.

Ces granulés dispersables, de densité apparente généralement comprise entre environ 0,3 et 0,6, ont une dimension de particules généralement comprise entre environ 0,15 et 2 mm et de préférence entre 0,3 et 1,5 mm.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique-, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 6 : Granulés dispersables

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 0,15 et 2 mm.

Exemple F 7 : Granulés dispersables

Dans un mélangeur, on mélange les constituants suivants :

* matière active (composé n° 1) 75 %
* agent mouillant (alkylnaphtalène sulfonate de sodium) 2 %
* agent dispersant (polynaphtalène sulfonate de sodium) 8 %
* charge inerte insoluble dans l'eau (kaolin) 15 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment herbicides, ces dernières étant sous la forme de poudres mouillables, ou de granulés ou suspensions aqueuses.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols ; les poudres mouillables (ou poudre à pulvériser) et les pâtes sont des compositions solides mais destinées à constituer des compositions liquides lors de l'application.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir, quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

Exemple F 8 :

* matière active 400 g/l
* dodécylbenzène sulfonate alcalin 24 g/l
* nonylphénol oxyéthylé à 10 molécules d'oxyde d'éthylène 16 g/l
* cyclohexanone 200 g/l
* solvant aromatique q.s.p 1 litre

Selon une autre formule de concentré émulsionnable, on utilise :

Exemple F 9 :

| | |
|---|---|
| * matière active | 250 g |
| * huile végétale époxydée | 25 g |
| * mélange de sulfonate d'alcoylaryle et d'éther de polyglycol et d'alcools gras | 100 g |
| * diméthylformamide | 50 g |
| * xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble: certaines matières solides organiques ou des sels minéraux peuvent être dissous dans le support pour aider à empêcher la sédimentation ou comme antigels pour l'eau.

A titre d'exemple, voici une composition de suspension concentrée :

Exemple F 10:

| | |
|---|---|
| * composé | 500 g |
| * phosphate de tristyrylphénol polyéthoxylé | 50 g |
| * alkylphénol polyéthoxylé | 50 g |
| * polycarboxylate de sodium | 20 g |
| * éthylène glycol | 50 g |
| * huile organopolysiloxanique (antimousse) | 1 g |
| * polysaccharide | 1,5 g |
| * eau | 327,5 g |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

La présente invention concerne aussi un procédé de désherbage (notamment de zones de culture dicotylédones ou monocotylédones) qui consiste à appliquer à la zone devant être désherbée, et/ou sur les plantes devant être détruites, une quantité efficace d'un composé selon l'invention, notamment de composé de formule (I), les plantes (ou mauvaises herbes) devant être détruites ou dont on veut empêcher la croissance pouvant être elle-même de type monocotylédones ou dicotylédones.

Les produits et compositions selon l'invention s'appliquent commodément sur la végétation et notamment sur les mauvaises herbes à éliminer lorsque celles-ci présentent un feuillage vert (application de postémergence).

On peut aussi utiliser un procédé de désherbage qui consiste à appliquer une quantité efficace d'un composé de formule (I) sur des zones ou terrains où l'on veut empêcher la pousse ou le développement de plantes n'ayant pas encore poussé (application de préémergence). On peut, dans ce cas, opérer de manière à ce que la culture soit semée avant ou après le traitement.

La dose d'application de matière active est généralement comprise entre 0,05 et 8 kg/ha, de préférence entre 0,4 et 4 kg/ha.

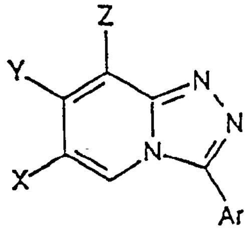

Formule (I)

Numérotation

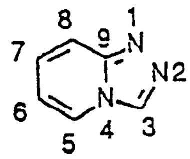

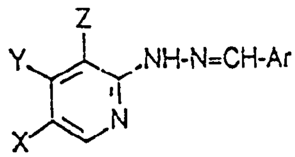

Formule (II)

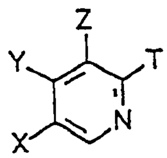

Formule (VIII)

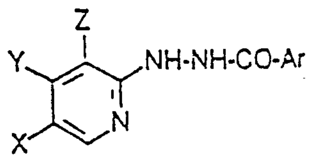

Formule (III)

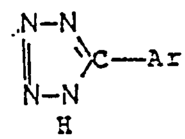

Formule (IX)

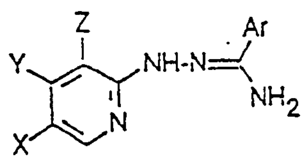

Formule (IV)

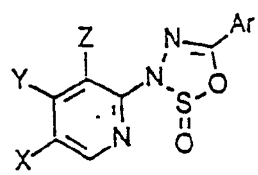

Formule (XI)

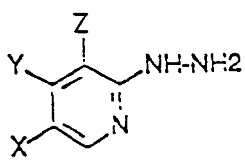

Formule (V)

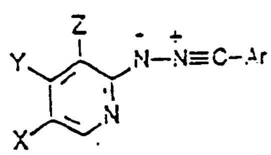

Formule (XII)

## Revendications

**1)** Composés de type triazolopyridine caractérisés en ce qu'ils ont la formule (I)

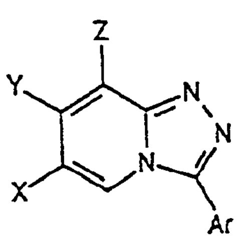

Formule (I)

dans laquelle :

* X , Y et Z représentent un atome d'hydrogène ou un atome d'halogène ou un groupe alkyle ou haloalkyle ou alkoxy, l'un au moins des radicaux X, Y ou Z ayant une signification autre que l'atome d'hydrogène,

* Ar représente un groupe phényle, éventuellement mono- ou poly- substitué par un groupe alkyle inférieur ou alkoxy inférieur ou alkylthio inférieur ou phényle ou phénoxy ou un atome d'halogène, de préférence le chlore ou le fluor ; ou un hétérocycle Het,

* Het représente un hétérocycle à 5 ou 6 maillons et contenant un ou plusieurs hétéroatome(s) tel(s) que le soufre, l'azote ou l'oxygène, cet hétérocycle étant éventuellement mono- ou poly-substitué par un groupe alkyle inférieur ou alkoxy inférieur ou alkylthio inférieur ou un atome d'halogène, de préférence le chlore ou le fluor,

ainsi que les sels acceptables en agriculture des composés définis ci-avant,

* à l'exclusion des composés tels que

** simultanément Y représente un groupe méthyle, X et Z représentent l'atome d'hydrogène, Ar représente un groupe 3-chlorophényle ou 4-chlorophényle ou 3-pyridyle ou 3,4,5-triméthoxyphényle ; ou bien

** simultanément X représente l'atome de chlore, Y et Z représentent l'atome d'hydrogène, Ar représente un groupe 2-pyridyle ou 4-pyridyle.

**2)** Composés selon la revendication 1, caractérisés en ce que X est autre que l'atome de chlore, et/ou que, lorsque Ar représente un groupe phényle substitué, les substituants se trouvent en position ortho ou para.

**3)** Composés selon l'une des revendications 1 ou 2, caractérisés en ce que :

Het est choisi parmi les groupes thiényles (de préférence 2- et 3- thiényle), thiazolyles (de préférence 2- et 4- thiazolyle), pyridyles (de préférence 2- pyridyle), pyrrolyles (de préférence 2-pyrrolyle), thiadiazolyles (de préférence 5-thiadiazolyles).

**4)** Composés selon l'une des revendications 1 à 3, caractérisés en ce que Ar est monosubstitué et/ou que Het représente un radical thiényle.

**5)** Composés selon l'une des revendications 1 à 4, caractérisés en ce que :

un seul ou deux des trois radicaux X, Y ou Z représente un radical autre que l'atome d'hydrogène,

lorsque X, Y ou Z sont un atome d'halogène, il s'agit de l'atome de chlore ou de brome,

lorsque X, Y ou Z sont un radical au moins partiellement hydrocarboné, ce radical a de préférence de 1 à 4 atomes de carbone, et plus préférentiellement encore 1 atome de carbone.

**6)** Composés selon l'une des revendications 1 à 5, caractérisés en ce que Z est autre que l'atome d'hydrogène.

**7)** Composés selon la revendication 6, caractérisés en ce que X et Y sont l'atome d'hydrogène.

**8)** Composés selon l'une des revendications 1 à 7, caractérisés en ce que Z est un atome de chlore ou un groupe méthyle.

**9)** Compositions herbicides caractérisées en ce qu'elles comportent 0,05 à 95 % en poids d'une matière active selon l'une des revendications 1 à 8 en combinaison avec un ou plusieurs supports solides ou liquides acceptables en agriculture et/ou agents tensio actifs acceptables en agriculture.

**10)** Procédé de désherbage caractérisé en ce qu'il consiste à appliquer, à la zone devant être désherbée et/ou sur les plantes devant être détruites, une quantité efficace d'un composé selon l'une des revendications 1 à 8 ou d'une composition selon la revendication 9. ,

**11)** Composés utilisables notamment comme intermédiaires pour la préparation de composés selon la revendication 1 caractérisés en ce qu'ils ont l'une des formules (II), (IV), (VIII), (XI) ou (XII)

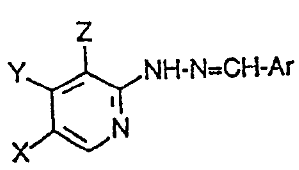

Formule (II)

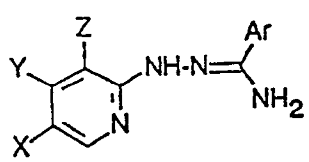

Formule (IV)

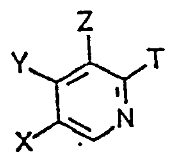

Formule (VIII)

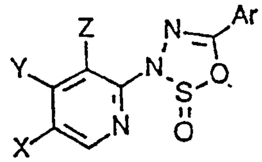

Formule (XI)

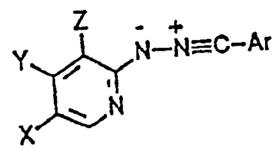

Formule (XII)

dans lesquelles les divers symboles ont les significations données dans l'une ou l'autre des revendications 1 à 8.

**12)** Procédé de préparation des composés selon l'une des revendications 1 à 8 caractérisé en ce qu'on fait réagir un composé de type arylidène 2-(pyrid-2'-yl)hydrazine ayant pour formule la formule (II) selon la revendication 11 avec un agent d'oxydation, selon une réaction d'oxydation cyclisante.

**13)** Procédé selon la revendication 12, caractérisé en ce que l'agent d'oxydation est choisi parmi les cations métalliques dérivés de métaux ayant plusieurs degrés d'oxydation et se trouvant à un degré d'oxydation supérieur, ou l'oxygène de l'air.

**14)** Procédé selon la revendication 12 caractérisé en ce que la réaction d'oxydation du composé de formule (II) est effectuée par addition d'un halogène, suivie d'une déshalogénation.

**15)** Procédé selon la revendication 12, caractérisé en ce que la réaction d'oxydation du composé de formule (II) est effectuée par addition d'un N-halogéno N-métallosulfonamidate (de préférence la chloramine T).

**16)** Procédé de préparation des composés selon l'une des revendications 1 à 8 caractérisé en ce qu'on déshydrate (déshydratation cyclisante) un composé de type 2-(aroylhydrazino)pyridine ayant pour formule la formule (III) dans laquelle les divers substituants ont la même signification que dans l'une ou l'autres des revendications 1 à 8.

**17)** Procédé selon la revendication 16 caractérisé en ce que l'on opère en présence d'un agent de déshydratation.

**18)** Procédé de préparation des composés selon l'une des revendications 1 à 8 caractérisé en ce qu'on opère une désamination cyclisante par chauffage d'un composé de type amidrazone ayant pour formule (IV) selon la revendication 11 dans laquelle les divers substituants ont même signification que dans l'une ou l'autre des revendications 1 à 8.

**19)** Procédé de préparation des composés selon l'une des revendications 1 à 8 caractérisé en ce qu'on fait réagir un composé de type hydrazine de formule (V) avec un composé de formule (VI) : $U_1$-C(=$W_1$)- Ar selon la réaction :

$$(V) + (VI) \text{ -----> } (I) + U_1H + W_1H_2$$

les divers radicaux de ces formules (V) et (VI) ayant les mêmes significations que dans l'une ou l'autre des revendications 1 à 8,

$W_1$ représente un atome d'oxygène ou groupe NH,

lorsque $W_1$ représente l'atome d'oxygène, alors $U_1$ représente un groupe hydroxyle ou alkoxy ou aroyloxy, ou un atome d'halogène,

lorsque $W_1$ représente un groupe NH, alors $U_1$ représente un groupe alkoxy ou alkylthio ou arylalkylthio.

**20)** Procédé de préparation des composés selon l'une des revendications 1 à 8 caractérisé en ce qu'on fait réagir un dérivé pyridinique de formule

(VIII)

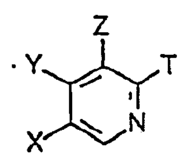

Formule (VIII)

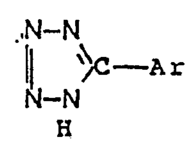

Formule (IX)

avec un composé tétrazolique de formule (IX) , formules dans lesquelles X, Y, Z et Ar ont la même signification que dans l'une ou l'autre des revendications 1 à 8 et T représente un atome d'halogène, de préférence le chlore.

**21)** Procédé de préparation d'arylidène 2-(pgrid-2'yl) hydrazines de formule (II) selon la revendication 11 caractérisé en ce qu'on fait réagir une 2-hydrazinopyridine de formule (V) selon la revendication 11 avec des

aldéhydes de formule Ar-CHO dans lesquelles les symboles X, Y, Z et Ar ont les significations indiquées dans l'une ou l'autre des revendications 1 à 6.

**22)** Procédé de préparation d'amidrazones de formule (IV) selon la revendication 11 caractérisé en ce qu'on fait réagir un dérivé de 2-hydrazinopyridine de formule (V) selon la revendication 11 selon le schéma :

$$(V) + Ar\text{-}C(=NH)\text{-}U_3 \rightarrow (IV) + U_3H$$

les divers radicaux de ces réactifs et produits de réaction ayant même signification que dans l'une ou l'autre des revendications 1 à 6, et $U_3$ est un radical alkoxy ou alkylthio.

**23)** Utilisation à titre d'herbicides des composés de formule (II).

Office européen des brevets

# RAPPORT DE RECHERCHE EUROPEENNE

Numero de la demande

EP 91 42 0024
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | FR-A-1549295 (C.F. BOEHRINGER UND SÖHNE GMBH)<br>* page 1; page 2, tableaux I et II *<br>--- | 1, 11, 12, 16, 18 | C07D471/04<br>C07D213/77<br>C07D257/04<br>C07D419/04<br>A01N43/90<br>//(C07D471/04, 249:00,221:00) |
| A | CHEMISCHE BERICHTE,<br>vol. 103, 1970,<br>pages 1918 - 1933; H. REIMLINGER et al.:<br>"1.5-Dipolare Cyclisierungen, III. Kondensierte Triazole aus konjugierten Nitriliminen, Struktur und Stabilität der Produkte aus ..."<br>* page 1921, schéma de réactions; composés 10b, 10c, 11b, 11c *<br>--- | 1, 3, 5, 11 | |
| A | CHEMICAL ABSTRACTS, vol. 84, no. 25, 21 juin 1976<br>Columbus, Ohio, USA<br>J. MORAGUES et al.: "Potential antiinflammatory agents. II. 7-Methyl-s-triazolo[4,3-a]pyridines"<br>page 568; ref. no. 180138S<br>& Farmaco, Ed. Sci. 1976, 31[2], 126-32<br>* abrégé *<br>--- | 1-4 | **DOMAINES TECHNIQUES RECHERCHES (Int. Cl.5 )** |
| A | CHEMICAL ABSTRACTS, vol. 83, no. 13, 29 septembre 1975<br>Columbus, Ohio, USA<br>page 561; ref. no. 114419N<br>& ES-A-400725<br>* abrégé *<br>--- | 1-4 | C07D213/00<br>C07D257/00<br>C07D291/00<br>C07D419/00<br>C07D471/00 |
| A | EP-A-0210648 (MERRELL DOW PHARMACEUTICALS INC.)<br>* page 2, ligne 10 - page 3, ligne 6; revendications 1, 7, 10 *<br>--- | 1, 16 | |
| A | DD-A-132463 (FISONS LTD.)<br>* revendications 1, 5 *<br>---<br>-/- | 9, 10 | |

Le présent rapport a été établi pour toutes les revendications

| Lieu de la recherche | Date d'achèvement de la recherche | Examinateur |
|---|---|---|
| BERLIN | 07 MAI 1991 | HASS C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la domande

EP 91 42 0024
Page 2

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.5 ) |
| A | CHEMISCHE BERICHTE, vol. 103, 1970, pages 1960 - 1981; H. REIMLINGER et al.: "Kondensierte Isochinoline, I. Synthesen von s-Triazolo[3,4-a]isochinolinen" * page 1960, schéma de réactions * ----- | 20 | |
| | | | DOMAINES TECHNIQUES RECHERCHES (Int. Cl.5 ) |

Le présent rapport a été établi pour toutes les revendications

| Lieu de la recherche | Date d'achèvement de la recherche | Examinateur |
|---|---|---|
| BERLIN | 07 MAI 1991 | HASS C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)